## Europäisches Patentamt

(19) **European Patent Office**

**Office européen des brevets**

(11) Publication number: **0 327 369**
**A2**

## (12) EUROPEAN PATENT APPLICATION

(21) Application number: **89301021.5**

(22) Date of filing: **02.02.89**

(51) Int. Cl.⁴: **C 12 N 15/00**
C 12 P 21/02, C 12 N 5/00,
C 12 Q 1/68, C 07 H 21/04,
G 01 N 33/50

(30) Priority: **02.02.88 US 151414**

(43) Date of publication of application:
**09.08.89 Bulletin 89/32**

(84) Designated Contracting States:
**AT BE CH DE ES FR GB GR IT LI LU NL SE**

(71) Applicant: **THE REGENTS OF THE UNIVERSITY OF CALIFORNIA**
**2199 Addison Street**
**Berkeley California 94720 (US)**

(72) Inventor: **Williams, Lewis T.**
**53 Cragmont Avenue**
**San Francisco California 94116 (US)**

**Escobedo, Jaime, E.**
**1467-7th Avenue**
**San Francisco California 94122 (US)**

(74) Representative: **Sheard, Andrew Gregory et al**
**Kilburn & Strode 30, John Street**
**London WC1N 2DD (GB)**

Claims for the following Contracting State: ES.

(54) **Human plalelet-derived growth factor receptor.**

(57) A DNA sequence encoding the human platelet-derived growth factor receptor (hPDGF-R) has now been isolated and sequenced. An expression construct comprises the sequence encodes a receptor that can be secreted or incorporated into the membrane of a mammalian cell. The incorporated receptor is functionally equivalent to the wild-type receptor, conferring a PDGF-sensitive mitogenic response on cells lacking the receptor. The construct can be used for enhancing PDGF response of cells, determining the regions involved in transducing the signal in response to PDGF binding, providing mutated analogs and evaluating drugs for their physiologic activity.

EP 0 327 369 A2

**Description**

## HUMAN PLATELET-DERIVED GROWTH FACTOR RECEPTOR

The present invention relates to growth factors and their receptors and, in particular, to human platelet-derived growth factor receptor.

Platelet-derived growth factor (PDGF) is a major mitogen for cells of mesenchymal origin. The protein is a 32 kDa protein heterodimer composed of two polypeptide chains, A and B, linked by disulfide bonds. In addition to the PDGF AB heterodimer, two homodimeric forms of PDGF, denoted AA and BB, have been identified. At the present time, there is no direct proof that the AA form of PDGF can bind to PDGF receptors.

The first event in PDGF-mediated mitogenesis is the binding of PDGF to its receptor at the cell membrane. This interaction triggers a diverse group of early cellular responses including activation of receptor tyrosine kinase, increased phosphatidylinositol turnover, enhanced expression of a group of genes, activation of phospholipase A2, changes in cell shape, increase in cellular calcium concentration, changes in intracellular pH, and internalization and degradation of bound PDGF. These changes are followed by an increase in the rate of proliferation of the target cells.

While the ability of a polypeptide to stimulate growth of a particular cell type in vitro does not prove that it serves the same function in vivo, the role of many growth factors on cells is being studied to attempt to determine the role that the factors play in the whole organism. In vitro, platelet-derived growth factor is a major polypeptide mitogen in serum for cells of mesenchymal origin such as fibroblasts, smooth muscle cells and glial cells. In vivo, PDGF circulates stored in the α granules of blood platelets and does not circulate freely in blood. During blood clotting and platelet adhesion, the granules are released, often at sites of injured blood vessels implicating PDGF in the repair of blood vessels. PDGF also stimulates migration of arterial smooth muscle cells from the medial to the intimal layer of the artery where they then proliferate as an early response to injury.

PDGF is being studied to determine how cell proliferation is controlled in the body. The growth factor has been implicated in wound healing, in atherosclerosis, and in stimulating genes associated with cancerous transformation of cells, particularly c-myc and c-fos. Therefore, PDGF agonists may be useful in promoting wound healing. PDGF antagonists may be useful in preventing atherosclerosis, in retarding blood vessel narrowing that occurs after cardiovascular intervention and in controlling cancerous proliferation.

The mouse PDGF receptor has been identified, purified (Daniel et al., Proc. Natl. Acad. Sci. USA (1985) 82:2684-2687), and sequenced (Yarden et al., Nature (1986) 323:226-232).

A DNA sequence encoding the human platelet-derived growth factor receptor (hPDGF-R) has now been isolated and sequenced. An expression construct comprising the sequence encodes a receptor that can be secreted or incoporated into the membrane of a mammalian cell. The incorporated receptor is functionally equivalent to the wild-type receptor, conferring a PDGF-sensitive mitogenic response on cells lacking the receptor. The construct can be used for enhancing PDGF response of cells, determining the regions involved in transducing the signal in response to PDGF binding, providing mutated analogs and evaluating drugs for their physiologic activity.

## DESCRIPTION OF SPECIFIC EMBODIMENTS

Methods for producing human platelet-derived growth factor (hPDGF-R) and nucleic and constructs for such production are provided as well as cells comprising the hPDGF-R where the composition and cells may find use in diagnosis, evaluation of drugs affecting the transduction of the hPDGF-R signal and in the treatment of diseases associated with hPDGF-R. Particularly, an expression construct encoding hPDGF-R is provided. The construct can be used to transfect cells providing a membrane-bound receptor that is functionally equivalent to the wild-type receptor, and conferring a PDGF-sensitive mitogenic response on cells lacking the receptor. The transfected cells can be used as a model for studying the PDGF-induced response of cells, determining the regions involved in transducing the signal in response to PDGF binding and evaluating drugs for their physiologic activity. The encoded receptor or its binding region also find use in evaluating PDGF agonists. Other utilities for the DNA sequence include use of fragments of the sequence as probes to detect deletions in the region of chromosome 5 where a number of growth-control related genes are clustered.

The nucleotide sequence of a cDNA sequence encoding hPDGF-R is set forth in Table 1 together with the deduced amino acid sequence of the receptor precursor. The sequence beginning at the amino acid numbered 1 corresponds to the amino terminus of human PDGF-R. The first 32 amino acids (designated -32 to -1) encode the signal peptide sequence. The dark bar underlines the transmembrane sequence (amino acid residues 500 to 524). Potential N-glycosylation sites are indicated by a line. The polyadenylation site in the 3′ end of the cDNA has been underlined.

CGCTGGCTGCTGGCAGCAGAGTGACTGCCCGCCCTATCTGGGACCCAGGATCGCTCTGTGAGCAACTTGGAGCCAGAGAGGAGATCAACAAGGAGGAGGAGAGAGCCGGCCCCCTCAGCC
CTGCTGCCCAGCAGCAGCCTGTGCTCGCCCTGCCCAACGCAGACAGCCAGACCCAGGGCGGCCCCCTCTGGCGGCTCTGCTCCTCCGAAGATGCTTGGGGGAGTGAGGCGACATGGGGCCG
.CTCCTCTCCCCTACAGCAGCCCCCTTCCTCCATCCCTCTGTTCTCCTGAGCCTTCAGGAGCCTGCACCAGTCCTGCCTGTCCTTCTACTCAGCTGTTACCCACTCTGGGACCAGCAGTC

TABLE 1

EP 0 327 369 A2

3

```
                                                                                           -32      -30
                                                                                          Het Arg Leu
TTTCTGATAACTGGGAGAGGGCAGTAAGGAGGACTTCCTGGAGGGGGTGACTGTCCAGAGCCTGGAACTGTGCCCACACCAGAAGCCATCAGCAGCAAGGACACC   ATG CGG CTT

                            -20                                                      -10                                     1
Pro Gly Ala Het Pro Ala Leu Ala Leu Lys Gly Glu Leu Leu Leu Leu Ser Leu Leu Leu Leu Leu Glu Pro Gln Ile Ser Gln Gly Leu
CCG GGT GCG ATG CCA GCT CTG GCC CTC AAA GGC GAG CTG CTG TTG CTG TCT CTC CTG TTA CTT CTG GAA CCA CAG ATC TCT CAG GGC CTG

                            10                                                       20                                      30
Val Val Thr Pro Pro Gly Pro Glu Leu Val Leu Asn Val Ser Ser Thr Phe Val Leu Thr Cys Ser Gly Ser Ala Pro Val Val Trp Glu
GTC GTC ACA CCC CCG GGG CCA GAG CTT GTC CTC AAT GTC TCC AGC ACC TTC GTT CTG ACC TGC TCG GGT TCA GCT CCG GTG GTG TGG GAA

                            40                                                       50                                      60
Arg Het Ser Gln Glu Pro Pro Gln Glu Het Ala Lys Ala Gln Asp Gly Thr Phe Ser Ser Val Leu Thr Leu Thr Asn Leu Thr Gly Leu
CGG ATG TCC CAG GAG CCC CCA CAG GAA ATG GCC AAG GCC CAG GAT GGC ACC TTC TCC AGC GTG CTC ACA CTG ACC AAC CTC ACT GGG CTA

                            70                                                       80                                      90
Asp Thr Gly Glu Tyr Phe Cys Thr Ile Asn Asp Ser Arg Gly Leu Glu Thr Asp Glu Arg Lys Arg Leu Tyr Ile Phe Val Pro Asp Pro
GAC ACG GGA GAA TAC TTT TGC ACC CAC AAT GAC TCC CGT GGA CTG GAG ACC GAT GAG CGG AAA CGG CTC TAC ATC TTT GTG CCA GAT CCC

                            100                                                      110                                     120
Thr Val Gly Phe Leu Pro Asn Asp Ala Glu Glu Leu Phe Ile Phe Leu Thr Glu Ile Thr Glu Ile Thr Ile Pro Cys Arg Val Thr Asp
ACC GTG GGC TTC CTC CCT AAT GAT GCC GAG GAA CTA TTC ATC TTT CTC ACG GAA ATA ACT GAG ATC ACC ATT CCA TGC CGA GTA ACA GAC

                            130                                                      140                                     150
Pro Gln Leu Val Val Thr Leu His Glu Lys Lys Gly Asp Val Ala Leu Pro Val Pro Tyr Asp His Gln Arg Gly Phe Ser Gly Ile Phe
CCA CAG CTG GTG GTG ACA CTG CAC GAG AAG AAA GGG GAC GTT GCA CTG CCT GTC CCC TAT GAT CAC CAA CGT GGC TTT TCT GGT ATC TTT

                            160                                                      170                                     180
Glu Asp Arg Ser Tyr Ile Cys Lys Thr Thr Ile Gly Asp Arg Glu Val Asp Ser Asp Ala Tyr Tyr Val Tyr Arg Leu Gln Val Ser Ser
GAG GAC AGA AGC TAC ATC TGC AAA ACC ACC ATT GGG GAC AGG GAG GTG GAT TCT GAT GCC TAC TAT GTC TAC AGA CTC CAG GTG TCA TCC

                            190                                                      200                                     210.
Ile Asn Val Ser Val Asn Ala Val Gln Thr Val Val Arg Gln Gly Glu Asn Ile Thr Leu Met Cys Ile Val Ile Gly Asn Glu Val Val
ATC AAC GTC TCT GTG AAC GCA GTG CAG ACT GTG GTC CGC CAG GGT GAG AAC ATC ACC CTC ATG TGC ATT GTG ATC GGG AAT GAG GTG GTC
```

EP 0 327 369 A2

```
                            220                                          230                                          240
Asn Phe Glu Trp Thr Tyr Pro Arg Lys Glu Ser Gly Arg Leu Val Glu Pro Val Thr Asp Phe Leu Leu Asp Met Pro Tyr His Ile Arg
AAC TTC GAG TGG ACA TAC CCC CGC AAA GAA AGT GGG CGG CTG GTG GAG CCG GTG ACT GAC TTC CTC TTG GAT ATG CCT TAC CAC ATC CGC

                            250                                          260                                          270
Ser Ile Leu His Ile Pro Ser Ala Glu Leu Glu Asp Ser Gly Thr Tyr Thr Cys Asn Val Thr Glu Ser Val Asn Asp His Gln Asp Glu
TCC ATC CTG CAC ATC CCC AGT GCC GAG TTA GAA GAC TCG GGG ACC TAC ACC TGC AAT GTG ACG GAG AGT GTG AAT GAC CAT CAG GAT GAA

                            280                                          290                                          300
Lys Ala Ile Asn Ile Thr Val Val Glu Ser Gly Tyr Val Arg Leu Leu Gly Glu Val Gly Thr Leu Gln Phe Ala Glu Leu His Arg Ser
AAG GCC ATC AAC ATC ACC GTG GTT GAG AGC GGC TAC GTG CGG CTC CTG GGA GAG GTG GGC ACA CTA CAA TTT GCT GAG CTG CAT CGG AGC

                            310                                          320                                          330
Arg Thr Leu Gln Val Val Phe Glu Ala Tyr Pro Pro Pro Thr Val Leu Trp Phe Lys Asp Asn Arg Thr Leu Gly Asp Ser Ser Ala Gly
CGG ACA CTG CAG GTA GTG TTC GAG GCC TAC CCA CCG CCC ACT GTC CTG TGG TTC AAA GAC AAC CGC ACC CTG GGC GAC TCC AGC GCT GGC

                            340                                          350                                          360
Glu Ile Ala Leu Ser Thr Arg Asn Val Ser Glu Thr Arg Tyr Val Ser Glu Leu Thr Leu Val Arg Val Lys Val Ala Glu Ala Arg His
GAA ATC GCC CTG TCC ACG CGC AAC GTG TCG GAG ACC CGG TAT GTG TCA GAG CTG ACA CTG GTT CGC GTG AAG GTG GCA GAG GCT CGC CAC

                            370                                          380                                          390
Tyr Thr Met Arg Ala Phe His Glu Asp Ala Glu Val Gln Leu Ser Phe Gln Leu Gln Ile Asn Val Pro Val Arg Val Leu Glu Leu Ser
TAC ACC ATG CGG GCC TTC CAT GAG GAT GCT GAG GTC CAG CTC TCC TTC CAG CTA CAG ATC AAT GTC CCT GTC CGA GTG CTG GAG CTA AGT

                            400                                          410                                          420
Glu Ser His Pro Asp Ser Gly Glu Gln Thr Val Arg Cys Arg Gly Arg Gly Met Pro Gln Pro Asn Ile Ile Trp Ser Ala Cys Arg Asp
GAG AGC CAC CCT GAC AGT GGG GAA CAG ACA GTC CGC TGT CGT GGC CGG GGC ATG CCC CAG CCG AAC ATC ATC TGG TCT GCC TGC AGA GAC

                            430                                          440                                          450
Leu Lys Arg Cys Pro Arg Glu Leu Pro Pro Thr Leu Leu Gly Asn Ser Ser Glu Glu Glu Ser Gln Leu Glu Thr Asn Val Thr Tyr Trp
CTC AAA AGG TGT CCA CGT GAG CTG CCG CCC ACG CTG CTG TGG AAC AGT TCC GAA GAG GAG AGC CAG CTG GAG ACT AAC GTG ACG TAC TGG

                            460                                          470                                          480
Glu Glu Glu Gln Glu Phe Glu Val Val Ser Thr Leu Arg Leu Gln His Val Asp Arg Pro Leu Ser Val Arg Cys Thr Leu Arg Asn Ala
GAG GAG GAG CAG GAG TTT GAG GTG GTG AGC ACA CTG CGT CTG CAG CAC GTG GAT CGG CCA CTG TCG GTG CGC TGC ACG CTG CGC AAC GCT

                            490                                          500                                          510
Val Gly Gln Asp Thr Gln Glu Val Ile Val Val Pro His Ser Leu Pro Phe Lys Val Val Val Ile Ser Ala Ile Leu Ala Leu Val Val
GTG GGC CAG GAC ACG CAG GAG GTC ATC GTG GTG CCA CAC TCC TTG CCC TTT AAG GTG GTG GTG ATC TCA GCC ATC CTG GCC CTG GTG GTG

                            520                                          530                                          540
Leu Thr Ile Ile Ser Leu Ile Ile Leu Ile Met Leu Trp Gln Lys Lys Pro Arg Tyr Glu Ile Arg Trp Lys Val Ile Glu Ser Val Ser
CTC ACC ATC ATC TCC CTT ATC ATC CTC ATC ATG CTT TGG CAG AAG AAG CCA CGT TAC GAG ATC CGA TGG AAG GTG ATT GAG TCT GTG AGC
```

```
      550                       560                       570
Ser Asp Gly His Glu Tyr Ile Tyr Val Asp Pro Met Gln Leu Pro Tyr Asp Ser Thr Trp Glu Leu Pro Arg Asp Gln Leu Gly
TCT GAC GGC CAT GAG TAC ATC TAC GTG GAC CCC ATG CAG CTG CCC TAT GAC TCC ACG TGG GAG CTG CCG CGG GAC CAG CTG GGA

      580                       590                       600
Arg Thr Leu Ser Gly Ala Phe Gly Gln Val Val Glu Ala Thr Ile Gly Leu Ser Gln Ala Ser Ile Met Lys Val Ala Val
CGC ACC CTC TCT GGG GCC TTT GGG CAG GTG GTG GAG GCC ACG ATC GGA CTG AGC CAG GCC AGC ATC ATG AAA GTG GCC GTC

      610                       620                       630
Lys Met Leu Lys Ser Ser Thr Ala Arg Ser Glu Lys Gln Ala Leu Met Ser Glu Lys Ile Leu Gly Pro His Leu Asn
AAG ATG CTT AAA TCC AGT ACA GCC CGC AGC GAG AAG CAA GCC CTT ATG TCG GAG AAG ATC CTG GGG CCC CAC CTG AAC

      640                       650                       660
Val Val Asn Leu Leu Leu Gly Ala [Cys] Thr Lys Gly Pro Ile Tyr [Cys] Arg Tyr Gly Asp Leu Val Asp Tyr Leu
GTG GTC AAC CTG TTG CTG GGG GCC  TGC  ACC AAA GGA CCC ATC TAT  TGC  CGC TAC GGA GAC CTG GTG GAC TAC CTG

      670                       680                       690
His Arg Asn Lys Gln His Phe Leu Gln Ile His Ser Asp Pro Arg Arg Pro Ser Asn Ala Leu Glu Leu Tyr Val Pro Val Gly
CAC CGC AAC AAA CAG CAC TTC CTG CAG ATC CAC TCC GAC CCC CGC CGC CCC AGC AAT GCC CTG GAG CTC TAC GTT CCC GTT GGG

      700                       710                       720
Val Pro Leu Pro Ser Ile Val Ser Leu Thr Glu Ser Asp Gly Gly Tyr Met Ser Lys Asp Glu Ser Val Asp Tyr Val Pro
GTC CCC CTG CCC AGC ATC GTG TCC CTG ACC GAG AGC GAC GGG GGT TAC ATG AGC AAG GAC GAG TCG GTG GAC TAT GTG CCC

      730                       740                       750
Met Leu Asp Met Lys Gly Asp Val Lys Tyr Ala Asp Ile Glu Ser Ser Asn Tyr Met Ala Pro Tyr Asp Asn Tyr Val Pro Ser Ala Pro
ATG CTG GAC ATG AAA GGA GAC GTC AAA TAT GCA GAC ATC GAG TCC AGC AAC TAC ATG GCA CCT TAC GAT AAC TAC GTT CCC TCC GCC CCT

      760                       770                       780
Glu Arg Thr [Cys] Arg Ala Thr Leu Ile Asn Glu Ser Pro Val Leu Ser Tyr Met Asp Leu Val Gly Phe Ser Tyr Gln Val Ala Asn Gly
GAG AGG ACC  TGC  CGA GCA ACT TTG ATC AAC GAG TCT CCA GTG CTA AGC TAC ATG GAC CTC GTG GGC TTC AGC TAC CAG GTG GCC AAT GGC

      790                       800                       810
Met Glu Phe Leu Ala Ser Lys Asn [Cys] Val His Arg Asp Leu Ala Ala Arg Asn Val Leu Val Cys Glu Gly Lys Val Lys Ile [Cys]
ATG GAG TTT CTG GCC TCC AAG AAC  TGC  GTC CAC AGA GAC CTG GCG GCT CGG AAC GTG CTG GTC TGT GAA GGC AAG GTC AAG ATC  TCT

      820                       830                       840
Asp Phe Gly Leu Ala Arg Asp Ile Met Ile Ser Asn Tyr Ile Ser Gly Ser Thr Phe Leu Pro Leu Lys Trp Met Ala Pro Glu
GAC TTT GGC CTG GCC CGA GAC ATC ATG ATC TCC AAT TAC ATC TCC GGG AGC ACC TTC CTC CCT TTA AAG TGG ATG GCT CCG GAG

      850                       860                       870
Ser Ile Phe Asn Ser Leu Tyr Thr Thr Leu Ser Asp Val Trp Ser Phe Gly Ile Leu Leu Trp Glu Ile Phe Thr Leu Gly Gly Thr Pro
AGC ATC TTC AAC AGC CTG TAC ACC ACC CTG TCG GAC GTG TGG AGC TTC GGC ATC CTC CTG TGG GAG ATC TTC ACC CTG GGC GGC ACC CCT
```

5

```
                               880                                            890                                            900
Tyr Pro Glu Leu Pro Met Asn Glu Gln Phe Tyr Asn Ala Ile Lys Arg Gly Tyr Arg Met Ala Gln Pro Ala His Ala Ser Asp Glu Ile
TAC CCA GAG CTG CCC ATG AAC GAG CAG TTC TAC AAT GCC ATC AAA CGG GGT TAC CGC ATG GCC CAG CCT GCC CAT GCC TCC GAC GAG ATC

                               910                                            920                                            930
Tyr Glu Ile Met Gln Lys Cys Trp Glu Glu Lys Phe Glu Ile Arg Pro Pro Phe Ser Gln Leu Val Leu Leu Leu Glu Arg Leu Leu Gly
TAT GAG ATC ATG CAG AAG TGC TGG GAA GAG AAG TTT GAG ATT CGG CCC CCC TTC TCC CAG CTG GTG CTG CTT CTC GAG AGA CTG TTG GGC

                               940                                            950                                            960
Glu Gly Tyr Lys Lys Lys Tyr Gln Gln Val Asp Glu Glu Phe Leu Arg Ser Asp His Pro Ala Ile Leu Arg Ser Gln Ala Arg Leu Pro
GAA GGT TAC AAA AAG AAG TAC CAG CAG GTG GAT GAG GAG TTT CTG AGG AGT GAC CAC CCA GCC ATC CTT CGG TCC CAG GCC CGC TTG CCT

                               970                                            980                                            990
Gly Phe His Gly Leu Arg Ser Pro Leu Asp Thr Ser Ser Val Leu Tyr Thr Ala Val Gln Pro Asn Glu Gly Asp Asn Asp Tyr Ile Ile
GGG TTC CAT GGC CTC CGA TCT CCC CTG GAC ACC AGC TCC GTC CTC TAT ACT GCC GTG CAG CCC AAT GAG GGT GAC AAC GAC TAT ATC ATC

                               1000                                           1010                                           1020
Pro Leu Pro Asp Pro Lys Pro Glu Val Ala Asp Glu Gly Pro Leu Glu Gly Ser Pro Ser Leu Ala Ser Ser Thr Leu Asn Glu Val Asn
CCC CTG CCT GAC CCC AAA CCC GAG GTT GCT GAC GAG GGC CCA CTG GAG GGT TCC CCC AGC CTA GCC AGC TCC ACC CTG AAT GAA GTC AAC

                               1030                                           1040                                           1050
Thr Ser Ser Thr Ile Ser Cys Asp Ser Pro Leu Glu Pro Gln Asp Glu Pro Glu Pro Glu Pro Gln Leu Glu Leu Gln Val Glu Pro Glu
ACC TCC TCA ACC ATC TCC TGT GAC AGC CCC CTG GAG CCC CAG GAC GAA CCA GAG CCA GAG CCC CAG CTT GAG CTC CAG GTG GAG CCG GAG

                               1060                                           1070        1074
Pro Glu Leu Glu Gln Leu Pro Asp Ser Gly Cys Pro Ala Pro Arg Ala Glu Ala Glu Asp Ser Phe Leu AM
CCA GAG CTG GAA CAG TTG CCG GAT TCG GGG TGC CCT GCG CCT CGG GCT GAA GCA GAG GAT AGC TTC CTG TAG GGGGCTGGCCCCTACCCTGCCCTG
CCTGAAGCTCCCCCCCTGCCAGCACCCAGCATCTCCTGGCCTGGCCTGACCGGGCTTCCTGTCAGCCAGGCTGCCCTTATCAGCTGTCCCCTTCTGGAAGCTTTCTGCTCCTGACGTGTT
GTGCCCCAAACCCTGGGGCTGGCTTAGGAGGCAAGAAAACTGCAGGGGCCGTGACCAGCCCTCTGCCTCCAGGGAGGCCAACTGACTCTGAGCCAGGGTTCCCCCAGGGAACTCAGTTTT
CCCATATGTAAAATGGGAAAGTTAGGCTTGATGACCCAGAATCTAGGATTCTCTCCCTGGCTGACAGGTGGGGAGACCGAATCCCTCCCTGGGAAGATTCTTGGAGTTACTGAGGTGGTA
AATTAACTTTTTTTCTGTTCAGCCAGCTACCCCTCAAGGAATCATAGCTCTCTCCTCGACTTTATCCACCCAGGAGCTAGGGAAGAGACCCTAGCCTCCCTGGCTGCTGGCTGAGCTAGGG
CCTAGCCTTGAGCAGTGTTGCCTCATCCAGAAGAAGCCAGTCTCCTCCCTATGATGGCCAGTAAATGCGTTCCCTGGCCCGAGCTGGTCTGGGGCCATTAGGCAGCCTAATTAATGCTGG
AGGCTGAGCCAAGTACAGGACACCCCCAGCCTGCCAGCCCTTGCCCCAGGGCACTTGGAGCACACGCACCATAGCCAAGTCCTGTGTCCCTGTCCTTCAGGCCCATCAGTCCTGGGGCTTTTT
CTTTATCACCCTCAGTCTTAATCCATCCACCAGAGTCTAGAAGGCCAGACGGGCCCCGCATCTGTGATGAGAATGTAAATGTGCCAGTGTGGAGTGGCCACGTGTGTGTGCCAGTATATG
GCCCTGGCTCTGCATTGGACCTGCTATGAGGCTTTGGAGGAATCCCTCACCCTCTCTGGGCCTCAGTTTCCCCTTCAAAAAAATGAATAAGTCGGACTTATTAACTCTGATGCCTTGCCAG
CACTAACATTCTAGAGTATTCCAGGTGGTTGCACATTTGTCCAGATGAAGCAAGGCCATATACCCTAAACTTCCATCCTGGGGGTCAGCTGGGCTCCTGGGAGATTCCAGATCACACATC
ACACTCTGGGGACTCAGGAACCATGCCCCTTCCCCAGGCCCCCAGCCAAGTCTCAAGAACACAGCTGCACAGGCCTTGACTTAGAGTGACAGCCGGTGTCCTGGAAAGCCCCCAGCAGCTG
CCCCAGGACATGGGAAGACCACGGGACCTCTTTCACTACCCACGATGACCTCCGGGGGTATCCTGGGCAAAAGGGACAAAGAGGGCAAATGAGATCACCTCCTGCAGCCCACCACTCCAG
CACCTGTGCCGAGGTCTGCGTCGAAGACAGAATGGACAGTGAGGACAGTTATGTCTTGGAAAAGACAAGAAGCCTCAGAGTGGGTACCCCAAGAAGGATGTGAGAGGTGGGCGCTTTGGA
GGTTTGCCCCTCACCCACCAGCTGCCCCATCCCTGAGGCAGCGCTCCATGGGGGTATGGTTTTGTCACTGCCCAGACCTAGCAGTGACATCTCATTGTCCCCAGCCCAGTGGGCATTGGA
GGTGCCAGGGGAGTCAGGGTTGTAGCCAAGACGCCCGCACGGGGAGGGTTGGGAAGGGGGTGCAGGAAGCTCAACCCCTCTGGCACCAACCCTGCATTGCAGTTGGCACCTTACTTCCCT
GGGATCCCCAGAGTTGGTCCAAGGAGGGGAGAGTGGGTTCTCAATACGGTACCAAAGATATAATCACCTAGGTTTACAAATATTTTTAGGACTCACGTTAACTCACATTTATACAGCAGAA
ATGCTATTTTGTGATGCTGTTAAGTTTTTCTATCTGTGTACTTTTTTTTTAAGGGAAAGATTTTAATATTAAACCTGGTCTTCTCAAAAAAAAAAAAAAAAAAAAAAAAAAAAAAAAAA
```

The DNA compositions of this invention may be derived from genomic DNA or cDNA, prepared by synthesis or combinations thereof. The DNA compositions may include the complete coding region encoding hPDGF-R or fragments thereof of interest, usually comprising at least 8 codons (24 bp), more usually at least 12 codons, where one or more introns may be present. While for the most part the wild-type sequence will be employed, in some situation one or more mutations may be introduced, such as deletions, substitutions or insertions resulting in changes in the amino acid sequence or providing silent mutations. The genomic sequence will usually not exceed 50 kbp, more usually not exceed about 10 kbp, preferably not greater than 6 kbp.

The DNA fragment encoding hPDGF-R finds use to isolate DNA encoding PDGF receptors of other species which share substantial homologies with hPDGF-R. Portions of the DNA fragment having at least about 10 nucleotides, usually at least about 20 nucleotides, and fewer than about 6 knt (kilonucleotides), usually fewer than about 0.5 knt, from a DNA sequence encoding hPDGF-R find use as probes. The probes can be used to determine whether mRNA encoding hPDGF-R is present in a cell.

Additionally, the human PDGF receptor gene is located at a site on chromosome 5 where a number of growth control related genes are clustered. At least one genetic disease, 5q minus syndrome, has been shown to involve a deletion in this region. Fragments of the hPDGF-R gene sequence may be used as a marker to probe the structure of this important region of the genome and to diagnose genetic diseases associated with this area of the genome.

The DNA fragment or portions thereof can also be used to prepare an expression construct for hPDGF-R. The construct comprises a DNA sequence encoding hPDGR-R under the transcriptional control of the native or other than the native promoter. Usually the promoter will be a eukaryotic promoter for expression in a mammalian cell, where the mammalian cell may or may not lack PDGF receptors. In cases where one wishes to expand the DNA sequence or produce the receptor protein or fragments thereof in a prokaryotic host, the promoter may also be a prokaryotic promoter. Usually a strong promoter will be employed to provide for high level transcription and expression.

The expression construct may be part of a vector capable of stable extrachromosomal maintenance in an appropriate cellular host or may be integrated into host genomes. The expression cassette may be bordered by sequences which allow for insertion into a host, such as transposon sequences, lysogenic viral sequences, or the like. Normally, markers are provided with the expression cassette which allow for selection of host containing the expression cassette. The marker may be on the same or a different DNA molecule, desirably the same DNA molecule.

In mammalian cells, the receptor gene itself may provide a convenient marker. However, in prokaryotic cells, markers such as resistance to a cytotoxic agent, complementation of a auxotrophic host to prototrophy, production of a dectectable product, etc. will be more convenient.

The expression construct can be joined to a replication system recognized by the intended host cell. Various replication systems include viral replication systems such as retroviruses, simian virus, bovine papilloma virus, or the like. In addition, the construct may be joined to an amplifiable gene, e.g., DHFR gene, so that multiple copies of the PDGF-R gene may be made.

Introduction of the construct into the host will vary depending upon the particular construction. Introduction can be achieved by any convenient means, including fusion, conjugation, transfection, transduction, electroporation, injection, or the like, as amply described in the scientific literature. The host cells will normally be immortalized cells, that is cells that can be continuously passaged in culture. For the most part, these cells may be convenient mammalian cell line which is able to express PDGF-R and where desirable, process the polypeptide so as to provide a mature polypeptide. By processing is intended glycosylation, ubiquitination, disulfide bond formation, or the like. Usually the host will be able to recognize the signal sequence for inserting hPGDF-R into the membrane of the cell. If secretion is desired, the transmembrane locater sequence may be deleted or mutated to prevent membrane insertion of the protein.

A wide variety of hosts may be employed for expression of the peptides, both prokaryotic and eukaryotic. Useful hosts include bacteria, such as E. coli, yeast, filamentous fungus, immortalized mammalian cells, such as various mouse lines, monkey lines, Chinese hamster ovary lines, human lines, or the like. For the most part, the mammalian cells will be immortalized cell lines. In some cases, the cells may be isolated from a neoplastic host, or wild-type cells may be transformed with oncogenes, tumor causing viruses, or the like.

Under many circumstances, it will be desirable to transfect mammalian cells which lack a PDGF receptor where the signal sequence directs the peptide into the cell membrane. Lymphocytes and cardiac myocytes are primary cells which lack a receptor. Also, Chinese hamster ovary cells (CHO), epithelial cells lines and a number of human tumor cell lines lack PDGF receptors.

Transfected cells find use as a model for studying cellular responses to PDGF. For controlled investigation, mammalian cells which lack a PDGF recep tor can be transfected with an expression construct comprising a DNA sequence encoding hPDGF-R. Cells are produced that encode a receptor that is functionally equivalent to the wild-type receptor and confer an PDGF-sensitive mitogenic response on the cell. In this way, the binding properties of the naturally-occurring PDGF may be analyzed, fragments tested as well as synthetic compounds both proteinaceous and non-proteinaceous. As demonstrated in the Experimental section, transfected cells were used to determine that the AA form of PDGF activates the receptor tyrosine kinase.

In addition to studying PDGF-mediated mitogenesis, the transfected cells can be used to evaluate a drug's ability to function as a PDGF agonist or antagonist. In particular, transfected cells can be contacted with the test drug, and the amount of receptor tyrosine kinase activation or the rate of DNA synthesis can be

7

determined in comparison to control cells in the presence or absence of PDGF, or analogs thereof of known activity.

The hPDGF-R protein expressed by transfected cells also finds use. If the peptide is secreted, the peptide may be isolated from the supernatant in which the expression host is grown. If not secreted, the peptide may be isolated from a lysate of the expression host. The peptide may then be isolated by convenient techniques employing HPLC, electrophoresis, gradient centrifugation, affinity chromatography, particularly using PDGF, etc., to provide a substantially pure product, particularly free of cell component contaminants.

The receptor protein or amino acids beginning at about 33 through about 500 of the amino terminal sequence of the receptor which form the external domain, binding portion of the receptor protein find use to affinity purify PDGF. The external domain can also be used affixed to a solid substrate or free in solution to determine drugs useful as PDGF agonists and antagonists.

The protein or the intracellular portion of the protein, beginning at about amino acid 525 through the carboxy terminal amino acid of hPDGF-R, also find use as an enzyme having tyrosine kinase activity. Additionally, amino acids 1 through 32 of the amino terminal sequence of the receptor comprise a signal sequence which directs the structural protein through the membrane of a transfected cell. The signal sequence can be used with hPDGF-R, but also finds use with other proteins.

Peptides or portions thereof may also be used for producing antibodies, either polyclonal or monoclonal. Antibodies are produced by immunizing an appropriate vertebrate host, e.g. mouse, with the peptide by itself, or in conjunction with a conventional adjuvant. Usually two or more immunizations will be involved, and the blood or spleen will be harvested a few days after the last injection.

For polyclonal antisera, the immunoglobulins may be precipitated, isolated and purified, including affinity purification. For monoclonal antibodies, the splenocytes normally will be fused with an immortalized lymphocyte, e.g. a myeloid line, under selective conditions for hybridomas. The hybridomas may then be cloned under limited dilution conditions and their supernatants screened for antibodies having the desired specificity. Techniques for producing antibodies are well known in the literature and are exemplified by U.S. Patent Nos. 4,381,292, 4,451,570 and 4,618,577.

## EXPERIMENTAL

### Screening of Human Kidney λGT11 cDNA Library and Human Placenta λGT10 cDNA Library

A full-length DNA sequence encoding the mouse PDGF receptor (mPDGF-R) protein was used as a probe to screen 250,000 plaques of a human kidney cDNA library. Nick translation was used to prepare a probe with specific activity of $12 \times 10^8$ cpm per $\mu$g. The filters were incubated with the probe ($10^5$ cpm per ml) in hybridization buffer containing 30% formamide, 1X Denhardt's solution, 5X SSC, 0.02M sodium phosphate pH 6.5 and 500 $\mu$g per ml of salmon sperm DNA. After 14 hr. of hybridization at 40°C, the filters were washed four times at 55°C with 0.2X SSC and 0.1% SDS and two additional times at 65°C with 0.2X SSC. The filters were then air dried and exposed for 16 hrs.

Ten positive clones were obtained which were rescreened with the full-length mPDGF-R probe. Individual clones were isolated and analyzed by restriction analysis using EcoRI endonuclease. The clone containing the largest insert (2.3 kb), designated clone HK-6, was further characterized and sequenced using dideoxy terminators. Clone HK-6 contained the receptor sequence from nucleotide 3554 to nucleotide 5691 plus nine bases from the poly A tail.

A nick-translated probe, prepared from the 2.3 kb HK-6 DNA, was used to screen 250,000 plaques of a human placenta cDNA library. This screening was performed at high hybridization stringency (50% formamide in the hybridization buffer described above). The filters were incubated with $5 \times 10^5$ cpm per ml of probe for 14-16 hrs. at 42°C. The filters were then washed at 65°C in 0.1% SSC and 0.1% SDS four times.

After secondary screening with the HK-6 probe, seven clones were selected and analyzed by restriction digestion with EcoRI endonuclease. A clone (HP-7) that contained a 4.5 kb insert was selected and characterized. The sequence of that clone is described in Table 1.

### Construction of Expression Vector

The 4.5 kb DNA fragment containing the complete coding sequence for the human PDGF receptor was isolated from the HP-7 clone EcoRI digestion. The gel purified fragment was cloned into the EcoRI site in the polylinker region of SV40 expression vector PSV7C. The pSV7d expression vector (provided by P. Luciw, University of California, Davis) was a pML derivative containing the SV40 early promoter region (SV40 nucleotides 5190-5270), a synthetic polylinker with restriction sites for EcoRI, SmaI, XbaI, and SalI followed by three translation terminator codons (TAA) and the SV40 polyadenylation signal (SV40 nucleotides 2556-2770) (Truett et al., DNA (1984) 4333-349). The EcoRI fragment containing the cDNA sequence obtained from the HP-7 clone was inserted at the EcoRI site of the pSV7d. In the resulting expression vector, the hPDGF-receptor gene was under transcriptional control of the SV40 promoter.

To ensure the proper orientation of the PDGF receptor insert (4.5 kb) with respect to the SV40 promoter, the positive clones were digested with SmaI endonuclease which cuts at position 573 of the receptor sequence and in the polylinker region of the expression vector.

Clones containing the receptor in the proper transcriptional orientation released a 4.0 kb insert in addition to the 3.2 kb fragment containing the expression vector plus 573 base pairs of the 5' end of the receptor. This plasmid, PSVRH5 was used to co-transfect cells with PSV2 neo plasmid that confers resistance to the antibiotic neomycin.

Cell Culture and Transfection of CHO Cells

CHO cell clone KI, obtained from the U.C.S.F. Tissue Culture Facility, were grown in Ham's F-12 media supplemented with 10% FCS (U.C.S.F. Tissue Culture Facility) and penicillin and streptomycin at 37°C in 5% $CO_2$/95% air.

pSVRH5 plasmid DNA (10 μg) and pSV2 neo (1 μg) were used to co-transfect $1 \times 10^6$ CHO cells by the calcium precipitation technique (Van der Eb et al., Methods Enzymology (1980) 65:826-839), with the addition of 10 μg chloroquinone diphosphate (CDP) to prevent degradation of the transfected DNA (Luthman and Magnusson, Nucl. Acid Res. (1983) 11:1295-1308). After 12 hrs. of exposure to the DNA, the cells were trypsinized and replated at 1:5 dilution. Twenty-four hours later, the antibiotic G418 (GIBCO), an analog of neomycin, was added to the cultures at a concentration of 400 μg/ml.

After two weeks under selection, independent colonies were picked and transferred to 24-well plates. Confluent cultures were assayed for the presence of PDGF receptor by immunoblot using anti-receptor antibodies. Colonies that were positive by this assay were single-cell cloned by end-limiting dilution.

Stable transfected clones were tested for the expression of the PDGF receptor message measured by RNA protection assays (Zinn et al., Cell (1983) 34:865-879) and for the presence of PDGF-stimulated receptor protein detected by antiphosphotyrosine antibodies (Frackelton et al., J. Biol. Chem (1984) 259:7909-7915).

Expression of hPDGF-R cDNA in CHO cells

CHO cells transfected with plasmid DNA containing the human receptor cDNA under the transcriptional control of the SV40 early promoter (CHO-HR5) and CHO cells transfected with a similar plasmid containing the mouse receptor cDNA (CHO-R18) were solubilized as previously described (Escobedo et al., J. Biol. Chem. (1988) 263:1482-1487). Extracts were analyzed by Western blot analysis using an antibody that specifically recognizes sequences in the receptor carboxy-terminal region as previously described (Escobedo et al., supra; Keating et al., ibid. (1987) 262:7932-7937). The 195 kDa protein is the mature receptor and the 160 kDa protein is the receptor precursor.

The expression of the receptor protein in the transfectants was demonstrated by using antibodies that recognize an intracellular sequence in the receptor. The clone that had the highest level of human receptor expression was chosen for further study. This transfectant had receptors that were labeled with $^{125}$I-PDGF as shown by the competitive binding studies described below.

Competitive Binding of the Different Forms of PDGF to its Receptor

The ability of the human recombinant AA and BB homodimers (Collins et al., Nature (1987) 328:621-624) to compete for the receptor sites and displace $^{125}$I-labeled PDGF was studied. Each homodimer was produced selectively by a yeast expression system (Brake et al.. Proc. Natl. Acad. Sci. (USA) (1984) 81:4642-4646) and was purified from yeast media that is devoid of other mesenchymal cell growth factors, thus avoiding the artifact of contamination by factors that might be present in mammalian expression systems.

BALB/c 3T3 cells and CHO transfectants (CHO-HR5) were incubated with $^{125}$I-PDGF (William et al., ibid (1982) 79:5067-5070) in the presence of increasing concentrations of AA and BB. Binding was carried out at 37°C for 45 min. in whole cell suspension. Unbound, radiolabeled PDGF was removed by centrifugation on a Ficoll gradient (Orchansky et al., J. Immunol (1986) 136:169-173). Non-specific binding, determined by incubating CHO cells with $^{125}$I-PDGF, accounted for 25 percent of the bound radioactivity.

The binding study demonstrated that the transfected cells can be used as a model to study the interaction of hPDGF with its receptor. In particular, this study demonstrated that the transfected human receptor was functionally identical to the native mouse receptor as indicated by the following results. Both AA and BB forms of PDGF competed for the $^{125}$I-PDGF labeled sites in the human receptor transfectants. For the transfected human receptor as well as the native mouse receptor, the BB form was of higher affinity than the AA form. When expressed in yeast, the AA form of PDGF may be processed aberrantly, giving it a lower affinity than the BB form for both the transfected cells and mouse 3T3 cells. The consistency of the pattern of competition shows that the AA form interacts with the transfected human receptor in the same way as it does with the native mouse receptor and demonstrates that these receptors are functionally identical.

Activation of the PDGF Receptor Tyrosine Kinase

The ability of recombinant AA and BB homodimers and of human partially purified AB PDGF to activate the receptor tyrosine kinase was studied. The yeast-derived AA and BB homodimeric forms and the platelet-derived AB form stimulated autophosphorylation of the transfected human receptor.

BALB/c 3T3 cells and CHO cells transfected with the human PDGF receptor cDNA (CC0-HR5) were incubated with increasing amounts of different forms of PDGF (AA, BB and AB). Following polyacrylamide-SDS electrophoresis, the phosphorylated receptor was identified by Western blot using an antiphosphotyrosine antibody (Wand, Mol. Cell. Biol. (1985) 5:3640-3643).

The receptor protein co-migrated with the 200 kDa molecular weight marker. The concentration of each form the was effective in stimulating autophosphorylation of the transfected human receptor was identical to the concentration that gave a similar autophosphorylation to the native mouse 3T3 receptor or the transfected mouse receptor.

These results show for the first time that the AA form of PDGF activates the receptor tyrosine kinase. Prior to use of the transfected cells, there was no demonstration that the AA form had hPDGF activity or that a single receptor was capable of recognizing all three forms of PDGF. Futher, the results demonstrate that the human cDNA encodes a receptor that is functionally equivalent to the wild-type receptor that is responsible for PDGF-stimulated tyrosine kinase activity in mouse 3T3 cells.

Thus, the transfected cells are useful models for studying PDGF-induced mitogenic reponses.

Rate of DNA Synthesis in CH0 Transfected Cells

BALB/c 3T3 cells and CH0 cells transfected with human PDGF receptor cDNA (CH0-HR5) were incubated with saturating concentrations of the three forms of PDGF. Untreated cells and cells treated with fetal calf serum (FCS) were used as negative and positive controls, respectively. The level of $^3$H-thymidine incorporation into DNA was determined by measuring the radioactivity of the acid-precipitable material as previously described (Escobedo, supra)

Transfection of CH0 cells with either human or mouse PDGF receptor conferred a PDGF-sensitive mitogenic response. All forms of PDGF stimulated DNA synthesis in both the human receptor transfectant and the mouse cells bearing the native receptor.

These data showed that the A chain homodimer and the B chain homodimer, like the AB platelet-derived form, were mitogens that can act through the receptor encoded by this human cDNA sequence. The mitogenic action of these forms of PDGF on mouse 3T3 cells and CH0 cells containing the transfected human receptor demonstrate that the responses were mediated by functionally identical receptors.

These studies were made possible by the availability of growth factor preparations devoid of contamination with other growth factors and by use of a receptor expression system in which all of the measured PDGF responses could be attributed to this single transfected receptor cDNA.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference. The invention now being fully described, it will be apparent to one of ordinary skill in the art that many changes and modifications can be made thereto without departing from the spirit or scope of the appended claims.

## Claims

1. A DNA fragment of fewer than about 50 kbp encoding human platelet-derived growth factor receptor (hPDGF-R).

2. A DNA fragment as claimed in Claim 1 wherein the fragment comprises a cDNA sequence of less than about 6 kbp.

3. A probe comprising a sequence consisting essentially of at least about 10 nt of the DNA sequence encoding hPDGF-R.

4. A probe as claimed in Claim 3 wherein the probe has from about 25 nucleotides to 100 nucleotides.

5. An expression construct for hPDGF-R comprising in the 5'-3' direction of transcription, a promoter and under the transcriptional regulation of the promoter, a DNA sequence encoding hPDGF-R joined to other than DNA naturally joined to the hPDGF-R-encoding DNA sequence.

6. An expression construct as claimed in Claim 5 wherein the promoter is a eukaryotic promoter.

7. An hPDGF-R fragment having PDGF receptor binding activity consisting essentially of amino acids beginning at about 33 through about 500 of the amino-terminal sequence of hPDGF-R.

8. A substantially pure preparation of hPDGF-R or physiologically active fragments thereof.

9. A cell transfected by an expression construct for hPDGF-R comprising in the 5'-3' direction of transcription, a promoter and under the transcriptional regulation of the promoter, a DNA sequence encoding hPDGF-R joined to other than DNA naturally joined to said hPDGF-R-encoding DNA sequence.

10. A method of evaluating a drug's ability to function as a hPDGF agonist or antagonist comprising:

(a) contacting mammalian cells with the drug which mammalian cells comprise an hPDGF receptor as a result of transfecting said cells with an expression construct comprising a DNA sequence encoding hPDGF-R with the drug; and

(b) determining the amount of a PDGF-induced response in the cells in comparison to untransfected cells or a drug providing a known response.

## Claims for the following Contracting State: ES

1. A process for the preparation of a DNA fragment of fewer than about 50 kbp encoding human platelet-derived growth factor receptor (hPDGF-R), the process comprising coupling successive nucleotides and/or ligating oligonucleotides.

2. A process as claimed in Claim 1 wherein the fragment comprises a cDNA sequence of less than about 6 kbp.

3. A process for the preparation of a probe comprising a sequence consisting essentially of at least about 10 nt of the DNA sequence encoding hPDGF-R, the process comprising coupling successive nucleotides and/or ligating oligonucleotides.

4. A process as claimed in Claim 3 wherein the probe has from about 25 nucleotides to 100 nucleotides.

5. A process for the preparation of an expression construct for hPDGF-R comprising in the 5'-3' direction of transcription, a promoter and under the transcriptional regulation of the promoter, a DNA sequence encoding hPDGF-R joined to other than DNA naturally joined to the hPDGF-R-encoding DNA sequence the process comprising coupling successive nucleotides and/or ligating oligonucleotides.

6. A process as claimed in Claim 5 wherein the promoter is a eukaryotic promoter.

7. A process for the preparation of an hPDGF-R fragment having PDGF receptor binding activity consisting essentially of amino acids beginning at about 33 through about 500 of the amino-terminal sequence of hPDGF-R, the process comprising coupling successive amino acid residues.

8. A process for the preparation of a substantially pure preparation of hPDGF-R or physiologically active fragments thereof, the process comprising coupling successive amino acid residues.

9. A process for the preparation of a transfected cell, the process comprising transfecting a cell by an expression construct for hPDGF-R comprising in the 5'-3' direction of transcription, a promoter and under the transcriptional regulation of the promoter, a DNA sequence encoding hPDGF-R joined to other than DNA naturally joined to said hPDGF-R-encoding DNA sequence.

10. A method of evaluating a drug's ability to function as a hPDGF agonist or antagonist comprising:

(a) contacting mammalian cells with the drug which mammalian cells comprise an hPDGF receptor as a result of transfecting said cells with an expression construct comprising a DNA sequence encoding hPDGF-R with the drug; and

(b) determining the amount of a PDGF-induced response in the cells in comparison to untransfected cells or a drug providing a known response.